# EUROPÄISCHE PATENTANMELDUNG

(11) **EP 0 704 220 A1**
(43) Veröffentlichungstag der Anmeldung: **03.04.1996**
(21) Anmeldenummer: 94115480.9
(22) Anmeldetag: 30.09.1994
(51) Int. Cl.: A61K 38/21

(54) **Verwendung von Interferon beta enthaltenden Arzneimitteln**

(71) Anmelder: Dr. Rentschler Biotechnologie GmbH, D-88471 Laupheim (DE)
(72) Erfinder: Brzoska, Josef Dr., D-88471 Laupheim (DE); Fierlbeck, Gerhard Dr., D-72076 Tübingen (DE)
(74) Vertreter: Schwabe - Sandmair - Marx

(57) **Zusammenfassung**

Die Erfindung betrifft die Verwendung von Interferon beta zur Herstellung von Arzneimitteln zur adjuvanten systemischen intramuskulären oder subcutanen Therapie von durch humanpathogene Papillomviren induzierten Erkrankungen in niedriger Dosierung.

## Beschreibung

Die Erfindung betrifft die Verwendung von Interferon beta zur Herstellung von Arzneimitteln zur adjuvanten systemischen intramuskulären oder subcutanen Therapie von durch humanpathogene Papillomviren induzierten Erkrankungen in niedriger Dosierung.

Interferone (IFNs) gehören zu den Regulatorproteinen des Immunsystems, den Cytokinen, und weisen eine antivirale, antiproliferative, zelldifferenzierende und immunmodulierende Wirkung auf. Aufgrund ihrer antigenen Eigenschaften werden 3 IFN-Familien unterschieden: IFN-α, IFN-β und IFN-γ. Unter diesen 3 IFN-Familien weist IFN-γ eine Reihe von Besonderheiten auf, weshalb es auch als Typ 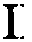 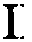 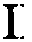 -IFN den beiden anderen, den Typ 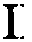 -IFNs, gegenübergestellt wird. Während die Bildung der Typ 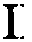 -IFNs durch Viren und doppelsträngige RNA ausgelöst wird, sind die Induktoren für IFN-γ Mitogene und spezifische Antigene. Entsprechend liegt die biologische Hauptfunktion der Typ 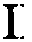 -IFNs in der antiviralen Wirkung, die von IFN-γ in der Immunregulation. Zudem bestehen zwischen den beiden IFN-Typen noch eine Reihe weiterer Unterschiede z.B. hinsichtlich Säurestabilität, Genlokalisation und Rezeptorbindung (Came und Carter 1984, Baron et al. 1991, Hündgen und von Eick 1991).

Außer den aus menschlichen Zellen gewonnenen (natürlichen) IFNs werden seit Anfang der 80er Jahre zunehmend gentechnologisch, mit Hilfe von Wirtszellen produzierte (rekombinante) IFNs in der Therapie von Erkrankungen des Menschen eingesetzt. Methoden zur Gewinnung natürlicher und rekombinanter IFNs sind in zahlreichen Publikationen, darunter vielen Patentanmeldungen, beschrieben (Übersichten bei Came und Carter 1984, Finter und Oldham 1985, Tabor 1986).

Für die Verwendung beim Menschen müssen rekombinante IFNs aufgrund ihrer Herstellung in Wirtszellen in hoher Reinheit (> 99 %) vorliegen, damit keine artfremden und daher z.T. toxischen Substanzen in den Arzneimitteln enthalten sind. In natürlichen IFN-Präparationen, die in der Regel nicht hochgereinigt werden, finden sich meist auch andere Cytokine (Came und Carter 1984, Finter und Oldham 1985).

Hinsichtlich der Pharmakokinetik weist IFN-β Besonderheiten auf. Während bei intramuskulärer (i.m.) oder subcutaner (s.c.) Verabreichung von IFN-α oder IFN-γ selbst in einer Dosierung von 1 x 10⁶ Internationale Einheiten (I.E.) noch Serumspiegel meßbar sind, ist bei gleicher Applikationsweise IFN-β erst bei einer Dosis von 3 - 9 x 10⁶ I.E. im Serum nachweisbar, woraus auf eine hohe Gewebsaffinität von IFN-β zum Ort seiner Applikation geschlossen werden kann (Koyama 1983, Finter und Oldham 1985, Hündgen und von Eick 1991, Wills 1990, Fierlbeck unveröffentlicht). IFN-β ist somit besonders für eine lokale Therapie geeignet (Hündgen und von Eick 1991). Eine systemische Behandlung mit IFN-β erfolgt daher häufig intravenös (i.v.) (Gebrauchsinformation Fiblaferon® der Fa. Rentschler, Laupheim, Deutschland; Gebrauchsinformation Feron® der Fa. Toray, Tokyo, Japan). Bei systemischer im. Applikation wird natürliches IFN-β für therapeutische Zwecke üblicherweise nur in Dosen von größer/gleich 2 x 10⁶ I.E. eingesetzt (Gebrauchsinformation Frone® der Fa. Serono, Rom, Italien). Eine Behandlung mit systemisch s.c. verabreichtem natürlichem IFN-β erfolgte bisher nur in Einzelfällen; für diese Applikationsweise fehlen daher allgemeingültige Therapieempfehlungen.

Rekombinantes IFN-β wurde in den bisher durchgeführten Dosisfindungsstudien i.v., i.m. und s.c. gegeben. Bei der Multiplen Sklerose beträgt die therapeutisch wirksame und empfohlene Tagesdosis 8 x 10⁶ I.E. i.m. bzw. 6 x 10⁶ I.E. s.c. (Gebrauchsinformation Betaseron® der Fa. Berlex, Richmond CA; Pressemitteilungen der Fa. Biogen, Cambridge Mass.).Bei der systemischen i.m. oder s.c. Applikation zur Behandlung verschiedener anderer Erkrankungen wie der chronisch myeloischen Leukämie (Aulitzky et al. 1993), der Hepatitis B und C (Irving Fox, Fa. Biogen, Cambrige Mass., persönliche Mitteilung) und der Condylomata acuminata (Gerd Gross, Universitäts-Hautklinik, Hamburg, persönliche Mitteilung; Robert Gerety, Fa. Biogen, Cambridge Mass., persönliche Mitteilung) betrug die niedrigste Tagesdosis 3 x 10⁶ I.E. Dementsprechend gibt es weder für natürliches noch für rekombinantes IFN-β Therapieempfehlungen, bei systemischer i.m. oder s.c. Applikation kleinere Tagesdosen als 2 x 10⁶ I.E. zu verwenden.

Humanpathogene Papillomviren (HPV) stellen eine heterogene Gruppe von DNA-Viren dar, die eine Reihe von epithelialen Tumoren (Warzen und Papillome) hervorrufen. Derzeit sind über 70 HPV-Typen bekannt. Zu den bekanntesten klinischen Erscheinungsbildern gehören die gemeinen Warzen (Verrucae vulgares), die Dornwarzen (Verrucae plantares) und die planen juvenilen Warzen (Verrucae planae juveniles), im Genitalbereich die Feigwarzen (Condylomata acuminata) und HPV-assoziierten Dysplasien des Gebärmutterhalses (cervicale intraepitheliale Neoplasien, Condylomata plana) (Kirby und Corey 1987, Cobb 1990, Gross et al. 1990, Lowy et al. 1994). Die Übertragung der genitalen HPV-Erkrankungen erfolgt in der Regel durch Geschlechtsverkehr. In England stellen die Condylomata acuminata (CA) die häufigste sexuell übertragene Viruserkrankung dar (Editorial Lancet 1991). Nach statistischen Erhebungen des amerikanischen Center for Disease Control (CDC) sind CA dreimal so häufig wie der genitale Herpes. So waren 1983 in den USA ca. 1 Million Patienten mit CA bei niedergelassenen Ärzten in Behandlung (Kirby und Corey 1987).

Defekte in der zellvermittelten Immunität fördern die HPV-Infektion und finden sich häufig bei Patienten mit rezidivierenden HPV-Erkrankungen (Kirby und Corey 1987, Cobb 1990, Editorial Lancet 1991). Die spontane Remissionsrate der HPV-Erkrankungen beträgt bei sonst gesunden Personen 20 % - 50 % während des ersten Jahres nach der Infektion. Es verwundert daher nicht, daß Placeboeffekte in der Therapie der HPV-Erkrankungen eine große Rolle spielen (Kirby 1988, Cobb 1990).

Ein eindeutiger wissenschaftlicher Wirkungsnachweis für eine bestimmte Behandlungsmethode ist daher nur durch randomisierte placebo-kontrollierte Studien möglich.

Die Therapie der HPV-Erkrankungen umfaßt vor allem chirurgische Maßnahmen wie Curettage, Elektrokauterisation, Kryo- und Laserchirurgie oder Touchierung mit keratolytischen und ätzenden Substanzen wie Salicylsäure, Podophyllin, Trichloressigsäure und 5-Fluorouracil (Kirby und Corey 1987, Cobb 1990, Gross et al. 1990, Cirelli und Tyring 1994). Insgesamt sind die Ergebnisse dieser Therapien aber wenig befriedigend, da die HPV-Erkrankungen häufig rezidivieren. Bei primären CA ist innerhalb von 3 Monaten mit einer Rezidivrate zwischen 20 und 40 % zu rechnen (Jensen 1985). Bei Patienten mit lange bestehenden und/oder rezidivierenden CA liegt die Rezidivrate in der Regel sogar über 50 % (Cirelli und Tyring 1994).

Aufgrund ihrer antiviralen, antiproliferativen und immunmodulierenden Eigenschaften scheinen die IFNs geeignete Substanzen für die Therapie der HPV-Erkankungen darzustellen (Cobb 1990). So verwundert es auch nicht, daß IFNs bereits in den 70er Jahren bei diesen Erkrankungen eingesetzt wurden. Die Applikationen erfolgten systemisch, topisch in einer Salbengrundlage und lokal durch intraläsionale Injektion. Übersichten über die seitdem durchgeführten, zahlreichen Studien geben Kirby and Corey 1987, Gross et al. 1990, Cirelli und Tyring 1994.

Die Wirksamkeit der IFNs ist bei der lokalen Therapie der CA zwar gesichert, für die Praxis aber nur von geringer Bedeutung, da diese Behandlungsform gegenüber anderen, konventionellen Therapieverfahren keine Vorteile bietet. Unbehandelte Warzen sprechen nicht bzw. nur unwesentlich an. Zudem sind die intraläsionalen Injektionen sehr schmerzhaft (Kirby 1988, Gross et al. 1990). Auf topische IFN-Präparationen sprechen nur kleine Warzen an (Brzoska 1994). Für CA im Analbereich sowie für großflächige Haut- bzw. Schleimhaut-Läsionen ist diese Applikationsform nicht geeignet.

In der systemischen Monotherapie von CA zeigten die bisher durchgeführten Studien uneinheitliche Ergebnisse. Placebo-kontrollierte Doppelblinduntersuchungen mit s.c. appliziertem rekombinantem IFN-α in einer Dosierung von 1.5, 3 und 9 x 10⁶ I.E. konnten die positiven Resultate offener Studien nicht bestätigen (Condylomata International Collaborative Study Group 1991, 1993). Mit natürlichem IFN-β wurden bisher nur wenige Studien durchgeführt. Hiervon wiesen zwei ein placebokontrolliertes Design auf. In beiden Studien wurde eine Tagesdosis von 2 x 10⁶ I.E. i.m. verwendet. Die Nebenwirkungen bei dieser Therapie waren gering. In den Verumgruppen zeigten signifikant mehr Patienten eine Abheilung ihrer CA als in den Placebogruppen (Schonfeld et al. 1984, Costa et al. 1988). Zu diesen positiven Ergebnissen ist allerdings anzumerken, daß in die Studien nur Patienten mit primären CA und einer Krankheitsdauer von 3 Wochen bis 6 Monaten aufgenommen wurden, nicht aber solche mit rezidivierenden älteren CA, die erfahrungsgemäß ein schlechteres Ansprechen auf die Behandlung mit IFNs zeigen (Gross et al. 1990, Fierlbeck et al. 1991, Cirelli und Tyring 1994). So konnte auch in einer offenen Studie bei Patienten mit alten CA in keinem Fall eine komplette Remission erzielt werden (Piccoli et al. 1989). Ergebnisse zu rekombinantem IFN-β liegen noch nicht vor. Für IFN-γ ließ sich in zwei placebo-kontrollierten Studien eine Wirksamkeit bei CA nachweisen (Gross et al. 1991). Nachteil der systemischen Monotherapie mit IFNs ist allerdings die z.T. lange Dauer der Behandlung bzw. Zeit bis zur Abheilung der Hauterscheinungen. Außerdem ist diese Therapieform nur bei etwa der Hälfte der Patienten erfolgreich. Von der überwiegenden Zahl der Dermatologen wird bislang daher die systemische Applikation der IFNs zur Behandlung der CA als ungeeignet angesehen (Kirby 1988).

In einer eigenen, doppelblinden, placebo-kontrollierten Studie haben wir für die systemische Therapie der CA rekombinantes IFN-β adjuvant verwendet, um die Rezidivrate zu verringern. Innerhalb von einer Woche nach chirurgischer Abtragung der CA wurde mit der IFN-β-Behandlung begonnen. Die Tagesdosis betrug im Gegensatz zu den bisher durchgeführten Behandlungen lediglich 1 x 10⁶ I.E. Die Applikationen erfolgten s.c. unter die Bauchhaut an 5 aufeinanderfolgenden Tagen und wurden nach einer Pause von 3 Wochen wiederholt (Gesamtdosis 10 x 10⁶ I.E.). Es wurden nur Patienten mit rezidivierenden CA aufgenommen. Innerhalb von 3 Monaten nach der chirurgischen Abtragung der CA wurde in beiden Gruppen die Rezidivrate bestimmt. Bereits bei einer Zwischenauswertung der Studie nach Aufnahme von 25 auswertbaren Patienten zeigte sich ein statistisch signifikanter Unterschied zwischen beiden Gruppen, d.h. die Rezidivrate der CA konnte durch die adjuvante Therapie mit systemisch s.c. verabreichtem IFN-β deutlich reduziert werden. Aufgrund der bisherigen Annahme über eine völlig unzureichende Wirkung von 1 x 10⁶ I.E. IFN-β nach s.c. Applikation war dieses Ergebnis nicht zu erwarten. Insbesondere überraschte, daß bei dieser geringen Dosis auch Patienten mit rezidivierenden CA ansprachen.

Außer dem oben beschriebenen Therapieregime können für die adjuvante systemische Therapie der HPV-Erkrankungen mit IFN-β auch andere Behandlungsschemata verwendet werden, wobei
die Dosis 0,5 - 1,5 x 10⁶ I.E.,
die Applikationsweise i.m. oder s.c.,
die Applikationsfrequenz 1 - 7 mal pro Woche und
die Dauer der Therapie 1 Tag bis 3 Monate
betragen kann.

Die zur i.m. oder s.c. Injektion vorgesehenen Formulierungen werden in Form von sterilen, wäßrigen Zubereitungen des aktiven Bestandteils, die vorzugsweise mit dem Blut des Empfängers isotonisch sind, hergestellt. Die zur Injektion hergerichteten Dosiseinheiten können in einzelnen sterilen Fläschchen z.B. als Lyophilisat in einer Menge von beispielsweise 0,5 x 10⁶, 1,0 x 10⁶ oder 1,5 x 10⁶ I.E. bezogen auf den reinen Wirkstoff IFN-β als Einzeldosis vorliegen, wobei der aktive Bestandteil unmittelbar vor Gebrauch durch Lösen mit Aqua ad injectabilia für die Applikation bereitgestellt wird. Als Formulierungspuffer kommen physiologisch verträgliche Puffer in Betracht, z.B. 0,1 M Natriumphosphat zusammen mit 0.05 M Natriumchlorid. Als Trägersubstanz eignet sich beispielsweise 0,5 - 30 mg/ml Humanserumalbumin für das Lyophilisat, bezogen auf die rekonstituierte Lösung.

### Literatur

Aulitzky WE, Peschel C, Desprès D, Aman J, Trautman P, Tilg H, Rudolf G, Hüttmann H, Obermeier J, Herold M, Huber C (1993): Divergent in vivo and in vitro antileukemic activity of recombinant interferon beta in patients with chronic-phase chronic myelogenous leukemia. Ann Hematol 67: 205-211
Baron S, Tyring SK, Fleischmann WR, Coppenhaver DH, Niesel DW, Klimpel GR, Stanton GJ, Hughes TK (1991): The interferons. Mechanisms of action and clinical applications. JAMA 266: 1375-1383
Brzoska J (1994) Topische Interferonpräparationen bei dermatologischen Erkrankungen. In: Gross G, Bröcker EB (eds) (1994): Interferon-Therapie in der Dermatologie. W Zuckschwerdt Verlag: München, Bern, Wien, New York, pp 1-9
Came PE, Carter WA (eds) (1984): Interferons and their applications. Springer Verlag: Berlin, Heidelberg, New York, Tokyo
Cirelli R, Tyring SK (1994): Interferons in human papillomavirus infections. Antiviral Res 24: 191-204
Cobb MW (1990): Human papillomavirus infection. J Am Acad Dermatol 22: 547-566
Condylomata International Colloborative Study Group (1991): Recurrent condylomata acuminata treated with recombinant interferon alfa-2a. A multicenter double-blind placebo-controlled clinical trial. JAMA 265: 2684-2687
Condylomata International Collobarative Study Group (1993): Recurrent condylomata acuminata treated with recombinant interferon alpha-2a. A multicenter double-blind placebo-controlled clinical trial: Acta Derm Venereol 73: 223-226
Costa S, Poggi MG, Palmisano L, Syrjänen S, Yliskosky M, Syrjänen K (1988): Intramuscolar β-interferon treatment of human papillomavirus lesions in the lower female genital tract. Cervix & l.f.g.t 6: 203-212
Editorial (1991): Persistent anogenital warts. Lancet 338: 1114-1116
Fierlbeck G, Breuninger H, Fierlbeck B, Rassner G (1991): Condylomata acuminata - lokale und systemische Interferontherapie. Hautarzt 42: 39-43
Finter NB, Oldham RK (eds) (1985): Interferon. In vivo and clinical studies. Elsevier: Amsterdam, New York, Oxford
Gross G, Jablonska S, Pfister H, Stegner HE (eds) (1990): Genital papillomavirus infections. Modern diagnosis and treatment. Springer: Berlin, Heidelberg, New York, London, Paris, Tokyo, Hong Kong, Barcelona
Gross G (1991) Recombinant interferon gamma in condylomata acuminata. JAMA 266: 2706
Hünden M, Eick H von (1991): Interferone. Grundlagen und Anwendung in Klinik und Praxis. Med Mo Pharm 14: 164-173
Jensen SL (1985): Comparison of podophyllin application with simple surgical excision in clearance and recurrence of perianal condylomata acuminata. Lancet ii: 1146-1148
Kirby P (1988): Interferon and genital warts: much potential, modest progress. JAMA 259: 570-572
Kirby P, Corey L (1987): Genital human papillomvirus infections. Infect Dis Clin North America 1: 123-143
Koyama Y (1983): Pharmacokinetics and clinical trials of HulFN-β in malignant tumors. In: Kishida T (ed) Interferons. Proceedings of the International Symposium on Interferons. ISIFN: Kyoto, Japan, pp 189-195
Lowy DR, Kirnbauer R, Schiller JT (1994): Genital human papillomavirus infection. Proc Natl Acad Sci 91: 2436-2440
Piccoli R, Santoro MG, Nappi C, Capodanno M, De Santis V, La Torre PC, Costa S, Montemagno U (1989): Vulvo-vagimal condylomatosis and relapse: combined treatment with electrocauterization and beta-interferon. Clin Exp Obst Gyn 16: 31-35
Schonfeld A, Nitke S, Schattner A, Wallach D, Crespi M, Hahn T, Levavi H, Yarden O, Shoham J, Doerner T, Revel M (1984): Intramuscular human interferon-β injections in treatment of condylomata acuminata. Lancet i: 1038-1042
Tabor JM (1986): Production-purification of interferons: recombinant technology. In: Stringfellow DA (ed) Clinical application of interferons and their inducers. Marcel Dekker, Inc: New York, Basel, pp 61-82
Wills RJ, (1990): Clinical pharmacokinetics of interferons. Clin Pharmacokinetic 19: 390-399

## Patentansprüche

1. Verwendung von Interferon beta zur Herstellung von Arzneimitteln zur adjuvanten systemischen intramuskulären (i.m.) oder subcutanen (s.c.) Behandlung von durch humanpathogene Papillomviren (HPV) induzierten Erkrankungen, enthaltend 0,5 bis 1,5 Millionen Internationale Einheiten (I.E.) als Tagesdosis, bezogen auf einen erwachsenen Patienten von etwa 60 kg Körpergewicht.

2. Verwendung von Interferon beta nach Anspruch 1 dadurch gekennzeichnet, daß das Interferon beta gentechnologisch hergestellt (rekombinant) ist.

3. Verwendung von Interferon beta nach Anspruch 1 dadurch gekennzeichnet, daß die zur Behandlung eingesetzten Arzneimittel zur subcutanen Applikation bestimmt sind.

4. Verwendung von Interferon beta nach Anspruch 1 dadurch gekennzeichnet, daß die zur Behandlung eingesetzten Arzneimittel als Tagesdosis 1 Million I.E. enthalten.

5. Verwendung von Interferon beta nach Anspruch 1 zur Herstellung von Arzneimitteln zur Behandlung genitaler HPV-Erkrankungen.

6. Verwendung von Interferon beta nach Anspruch 5 zur Herstellung von Arzneimitteln Behandlung von Condylomata acuminata.

7. Verwendung von Interferon beta nach Anspruch 6 zur Herstellung von Arzneimitteln Behandlung rezidivierender Condylomata acuminata.

8. Verwendung von Interferon beta nach den Ansprüchen 1-7, dadurch gekennzeichnet, daß die adjuvante systemische i.m. oder s.c. Behandlung nach der chirurgischen Abtragung erfolgt.

9. Verwendung von Interferon beta nach Anspruch 8, dadurch gekennzeichnet, daß die chirurgische Abtragung durch Maßnahmen wie Curettage, Elektrokauterisation, Kryo-und/oder Laserchirurgie erfolgt.

10. Verwendung nach den Ansprüchen 1-7, dadurch gekennzeichnet, daß die adjuvante systemische i.m. oder s.c. Behandlung nach Touchierung mit keratolytischen und ätzenden Substanzen wie Salicylsäure, Podophyllin, Trichloressigsäure und 5-Fluorouracil erfolgt.

11. Verwendung von Interferon beta nach den Ansprüchen 1-11, dadurch gekennzeichnet, daß die Applikationfrequenz der Tagesdosis zwischen 1 und 7 mal pro Woche beträgt.

12. Verwendung von Interferon beta nach Anspruch 11, dadurch gekennzeichnet, daß die Applikation von je 1 x 10⁶ I.E. s.c. an 5 aufeinanderfolgenden Tagen erfolgt.

13. Verwendung von Interferon beta nach Anspruch 12, dadurch gekennzeichnet, daß die Applikation an 5 aufeinanderfolgenden Tagen nach 3-wöchiger Pause wiederholt wird.

14. Verwendung von Interferon beta nach Anspruch 1-13, dadurch gekennzeichnet, daß die i.m. oder s.c. verabreichbare Injektionslösung neben NaCl einen pharmazeutisch üblichen Gehalt an Natriumphosphatpuffer sowie Humanserumalbumin aufweist.
